# EUROPEAN PATENT APPLICATION

(11) **EP 1 358 879 A1**
(43) Date of publication of application: **05.11.2003**
(21) Application number: 01270213.0
(22) Date of filing: 06.12.2001
(51) Int. Cl.: A61K 31/235, A61K 45/00, A61K 31/22, A61P 3/06, A61P 43/00

(54) **ANTILIPEMIC AGENTS**

(30) Priority: 13.12.2000 JP 2000379347
(71) Applicant: SHIONOGI & CO., LTD., Osaka-shi, Osaka 541-0045 (JP)
(72) Inventor: MIZUI, Takuji, c/o Shionogi & Co., Ltd., Osaka-shi, Osaka 553-0002 (JP); HARA, Seijiro, c/o Shionogi & Co., Ltd., Toyonaka-shi, Osaka 561-0825 (JP)
(74) Representative: Hayes, Adrian Chetwynd
(86) International application number: JP0110660
(87) International publication number: WO02047678

(57) **Abstract**

An antilipemic agent containing methyl 1-(3,4-dimethoxyphenyl)-3-(3-ethylvaleryl)-4-hydroxy-6,7,8-trimethoxy-2-naphthoate or a glucuronic acid conjugate thereof and an HMG-CoA reductase inhibitor.

## Description

### Technical Field

The present invention relates to an antilipemic agent containing a lignan analog and an HMG-CoA reductase inhibitor.

### Background Art

### Methyl

1-(3,4-dimethoxyphenyl)-3-(ethylvaleryl)-4-hydroxy-6,7,8-trimethoxy-2-naphthoate (hereinafter referred to as compound A) is a lignan analog possessing an antilipemic activity based on the inhibition of the re-absorption of bile acid in the small intestine (JP Patent publication (Kokai) 93/310634, corresponding USP 5,420,333, "Asunoshinyaku" 2000 year (Technomics), Life Sci. 1997, 60: PL365, J. Pharmacol. Exp. Ther. 1998, 284: 43, Arterioscler. Thromb. Vasc. Biol. 1998, 18: 1304). A glucuronic acid conjugate thereof (e.g., [1-0-{4-(3,4-dimethoxyphenyl)-2-(3-ethylpentanoyl)-5,6,7-trimethoxy-3-(methoxycarbo nylnaphthalene-1-yl}-β -D-glucopyranoside] uronic acid) is also known as possessing the antilipemic activity (JP Patent publication (Kokai) 97/241206).

In contrast, 3-hydroxy-3-methylglutaryl coenzyme (HMG-CoA) reductase inhibitor inhibits specifically HMG-CoA reductase, a rate-limiting enzyme in the cholesterol biosynthesis, so as to suppress the synthesis of cholesterol, thus being effective for the treatment of hypercholesterolemia, hyperlipoproteinemia, atherosclerosis and the like (Am. J. Med. 1998, 104(2A): 19S).

WO 98/40375 refers to an antilipemic agent containing a bile acid re-absorption inhibitor such as benzothiazepines and an HMG-CoA reductase inhibitor, without disclosing any concrete data of the antilipemic activity as expected upon the combination use of the both inhibitors.

Further, Arterioscler Thromb Vasc Biol. 1998; 18: 1304-1311 (Inhibition of Ileal Na+ / Bile Acid Cotransporter by S-8921 Reduces Serum Cholesterol and Prevents Atherosclerosis in Rabbits) reports that oral administration of compound A to rabbits increased the HMG-CoA reductase activity in the liver, without mentioning any concrete data of a therapeutic effect upon the combination use of an HMG-CoA reductase inhibitor.

Accordingly, it has been desired to develop a more effective method for the prevention or treatment of hyperlipemia using compound A or the like, as well as a pharmaceutical composition therefor.

### Disclosure of Invention

The present inventors have found that use of a HMG-CoA reductase inhibitor in combination with the above described compound A or a glucuronic acid conjugate thereof can lower the cholesterol concentration in blood more significantly than single use of the HMG-CoA reductase inhibitor, whereby to accomplish the present invention shown below.
(1) An antilipemic agent, which contains methyl
   1-(3,4'dimethoxyphenyl)-3'(3-ethylvaleryl)-4-hydroxy-6,7,8-trimethoxy-2-naphthoate, a glucuronic acid conjugate thereof, their salt, or a solvate thereof and an HMG-CoA reductase inhibitor.
(2) The antilipemic agent according to above (1), wherein the HMG-CoA reductase inhibitor is mevastatin, pravastatin, BMY-22089, colestrone, lovastatin, crilvastatin, dalvastatin, SC-45355, simvastatin, acitemate, BMS-180431, SQ-33600, CP-83101, BB-476, BMY-21950, L-669262, fluvastatin, itavastatin, glenvastatin, S-2468, cerivastatin, DMP-565, atorvastatin, bervastatin, rosuvastatin, carvastatin, U-20685, HBS-107, BAY-x-2678 or BAY-10-2987.
(3) The antilipemic agent according to above (1), wherein the HMG-CoA reductase inhibitor is pravastatin.
(4) An agent for enhancing the antilipemic effect of an HMG-CoA reductase inhibitor, which contains methyl
   1-(3,4-dimethoxyphenyl)-3-(3-ethylvaleryl)-4-hydroxy-6,7,8-trimethoxy-2-naphthoate, a glucuronic acid conjugate thereof, their salt or a solvate thereof.
(5) Use of methyl 1-(3,4-dimethoxyphenyl)-3-(3-ethylvaleryl)-4-hydroxy-6,7,8-trimethoxy-2-naphthoate, a glucuronic acid conjugates thereof, their salt or a solvate thereof for enhancing the antilipemic effect of an HMG-CoA reductase inhibitor.
(6) A preventive or therapeutic method for hyperlipemic disease, which comprises administrating methyl
   1-(3,4-dimethoxyphenyl)-3-(3-ethylvaleryl)-4-hydroxy-6,7,8-trimethoxy-2-naphthoate, a glucuronic acid conjugates thereof, their salt or a solvate thereof in combination with an HMG-CoA reductase inhibitor.

### Brief Description of Drawings

This graph shows a hypocholesterolemic effect in WHHL rabbits. The horizontal axis represents time (week) after the start of administration of a medicine and the vertical axis represents a change of the cholesterol level in the medicine-administered group compared with that of the control group.

### Best Mode for Carrying Out the Invention

A glucuronic acid conjugate of compound A (methyl 1-(3,4-dimethoxyphenyl)-3-(3-ethylvaleryl)-4-hydroxy-6,7,8-trimethoxy-2-naphthoate) means preferably [1-0-{4-(3,4-dimethoxyphenyl)-2-(3-ethylpentanoyl)-5,6,7-trimethoxy-3-(methoxycarbo nyl) naphthalene-1-yl}- β -D-glucopyranoside] uronic acid.

A salt of compound A or glucuronic acid conjugate thereof means preferably a pharmaceutically acceptable salt. Examples thereof include alkaline metal salts (e.g., Na, K), alkaline earth metal salts (e.g., Ca, Mg), amine salts (e.g., n-butylamine, sec-butylamine, i-propylamine, triethylamine, phenylamine, diphenylamine, N-phenyl-N-methylamine, pyridine, piperidine, morpholine, N-methylpiperidine, N-methyl morpholine). Examples of solvent of a solvate include alcohol and water.

In the present description, compound A, a glucuronic acid conjugate and a solvate thereof may be hereinafter collectively referred to as "lignan analog" .

Examples of HMG-CoA reductase inhibitor include those described in WO 98/40375, "Asunoshinyaku" (Technomics) and the like, and preferred are mevastatin, pravastatin, BMY-22089 (trans-6-[4,4-bis(4-fluorophenyl)-3-(1-methyl-1H-tetrazol-5-yl)-1,3-butadienyl]-tetrahydro-4-hydroxy-2H-pyran-2-one), colestrone, lovastatin, crilvastatin, dalvastatin, SC-45355, simvastatin, acitemate, BMS-180431 (trans-6(S)-[4,4-bis(4-fluorophenyl)-3-(1-methyl-1H-tetrazol-5-yl)-1,3-butadienyl]tetrahydro-4-hyd roxy-2H-pyran-2-one), SQ-33600 ((S)-4-[[[1-(4-fluorophenyl)-3-(1- methylethyl)-1H-indol-2-yl]ethynyl]hydroxyphosphinyl]-3-hydroxybutanoic acid, disodium salt), CP-83101 (methyl (3R,5S)-(E)-3,5-dihydroxy-9,9-diphenyl-6,8-nonadienoate), BB-476, BMY-21950 (sodium (±)-erythro-9,9-bis(4-fluorophenyl)-3,5-dihydroxy-8-(1-methyl-1H-tetrazol-5-yl)-6,8-nonadienoate), L-669262 (1,2,6,7,8,8a-hexahydro-3,7-dimethyl-6-oxo-8-[2-(tetrahydro-4-hydroxy-6-oxo-2H-pyra n-2-yl)ethyl]-1-naphthalenyl[1S-[1 α ,7 β ,8 β (2S*,4S*),8a β ]]-2,2-dimethyl-butanoic acid), fluvastatin, itavastatin, glenvastatin, S-2468, cerivastatin, DMP-565, atorvastatin, bervastatin, rosuvastatin (ZD-4522), carvastatin, U-20685 (17-hydroxyimino-1,3,5(10)-estratriene-3-ol), HBS-107, BAY-x-2678 or BAY-10-2987.

Preferred are pravastatin, lovastatin, simvastatin, fluvastatin, itavastatin, cerivastatin, atorvastatin, rosuvastatin (ZD-4522), HBS-107, BAY-x-2678, BAY-10-2987 and the like, more preferred are statin-type compounds having, as a side chain, 3,5-dihydroxycarboxylic acid or the lactone form, such as pravastatin, lovastatin, simvastatin, fluvastatin, itavastatin, cerivastatin, atorvastatin, rosuvastatin, esp. pravastatin and rosuvastatin.

The antilipemic agent of the present invention containing the above-described lignan analog and HMG-CoA reductase inhibitor may be in any formulation including e.g., granules, complex granules, powders, tablets, capsules, complex capsules. These formulations may contain an appropriate amount of well known medicinal additives such as binders, excipients, disintegrators, and dispersants.

Examples of the amount ratio of the above lignan analog and HMG-CoA reductase inhibitor includes, but not limited thereto: preferably compound A : HMG-CoA reductase inhibitor = 1:500 to 500:1, more preferably 1:50 to 50:1, and most preferably 1:10 to 10:1, by mol.

The above antilipemic agent can be preferably administrated orally. The daily dose for an adult is the lignan analog about 0.01 to 50 mg/kg, preferably about 0.1 to 30 mg/kg and HMG-CoA reductase inhibitor about 0.001 to 30 mg/kg, preferably about 0.01 to 10 mg/kg.

The combination use of the above lignan analog and HMG-CoA reductase inhibitor can bring more potent antilipemic effects than each single use. Thus, the composition of the present invention containing both is useful as a preventive or therapeutic agent for hyperlipemic disease.

The method for the above combination use may be, but not limited thereto, a simultaneous administration or time-lapse administration. The administered composition(s) may be single unit formulation or separated-type formulations. Their dose ratio is in accordance with the above mentioned one.

Further, the present invention provides an agent for enhancing the antilipemic effect of an HMG-CoA reductase inhibitor, which contains the above mentioned lignan analog, as well as use of the lignan analog for enhancing the antilipemic effect of an HMG-CoA reductase inhibitor. The dose ratio of the lignan analog per the HMG-CoA reductase inhibitor and each dose may be in accordance with the above mentioned one. Examples of the enhancing agent include a formulation enhancing the inhibitory activity itself of an HMG-CoA reductase inhibitor, as well as a formulation which can, even when possessing little or no such a direct enhancing effect to an HMG-CoA reductase inhibitory activity, consequently enhance the antilipemic effect of the co-used HMG·CoA reductase inhibitor.

Examples of the present invention are shown below.

### Formulation Example 1

Compound A (methyl1-(3,4-dimethoxyphenyl)-3-(3-ethylvaleryl)-4-hydroxy-6,7,8-trimethoxy-2- naphthoate) and pravastatin are mixed and granulated together with mannitol, carmellose calcium, and hydroxypropyl cellulose each in a proper amount, to prepare granules.

### Formulation Example 2

A glucuronic acid conjugate of compound A and lovastatin are mixed and granulated together with mannitol, carmellose calcium and hydroxypropyl cellulose each in a proper amount, to prepare granules.

### Formulation Example 3

A mixture of the granule of Formulation Example 1 or 2 and stearic acid is capsulated into a hard capsule to prepare capsules.

### Formulation Example 4

Compound A is mixed and granulated together with mannitol, carmellose calcium and hydroxypropyl cellulose each in a proper amount to prepare an agent for enhancing the effect of rosuvastatin.

### Test Example 1

WHHL rabbits, 24-weeks old were divided into the following groups.
1) non-treated control group (n=7; 3 males and 4 females)
2) group administered with Compound A 10 mg/kg/day (n=6; 2 males and 4 females)
3) group administered with pravastatin 3 mg/kg/day (n=6; 2 males and 4 females)
4) group co-administered with Compound A and pravastatin (n=7; 3 males and 4 females)

The test was conducted over 8 weeks including 6 weeks of administration and next 2 weeks of drug withdrawal, by collecting blood at intervals to measure the concentration of serum cholesterol. Any drug was administered as a mixture with food.

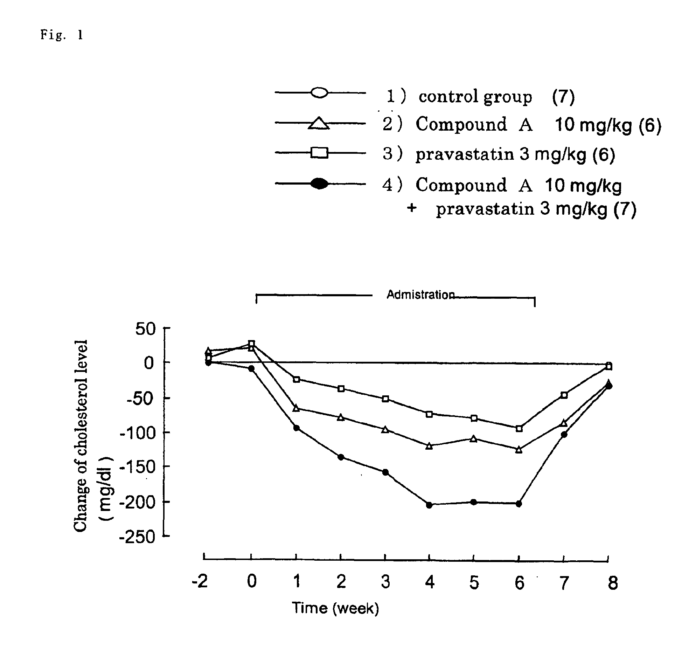

The results are shown in Fig. 1, wherein each line shown of ○, Δ, □ and ● corresponds to the above groups 1) to 4), respectively.

The co-administration of compound A with pravastatin more significantly lowered the cholesterol level than single use of pravastatin. In particularly, at 4 weeks after the start of co-administration, the cholesterol lowering was 3 times as strong compared with that by the single use of pravastatin.

### Test example 2

Each formulation of Formulation Examples 1 to 3 is administered to rabbits according to Test Example 1, to confirm the cholesterol-lowering effect.

### Industrial Applicability

The combination use of an HMG-CoA reductase inhibitor and a lignan analog of the present invention is effective for the prevention or treatment of hyperlipemic disease.

## Claims

1. An antilipemic agent, which contains methyl 1-(3,4-dimethoxyphenyl)-3-(3-ethylvaleryl)-4-hydroxy-6,7,8-trimethoxy-2-naphthoate, a glucuronic acid conjugate thereof, their salt, or a solvate thereof and an HMG-CoA reductase inhibitor.

2. The antilipemic agent according to claim 1, wherein the HMG-CoA reductase inhibitor is mevastatin, pravastatin, BMY-22089, colestrone, lovastatin, crilvastatin, dalvastatin, SC-45355, simvastatin, acitemate, BMS-180431, SQ-33600, CP-83101, BB-476, BMY-21950, L-669262, fluvastatin, itavastatin, glenvastatin, S-2468, cerivastatin, DMP-565, atorvastatin, bervastatin, rosuvastatin, carvastatin, U-20685, HBS-107, BAY-x-2678 or BAY-10-2987.

3. The antilipemic agent according to claim 1, wherein the HMG-CoA reductase inhibitor is pravastatin.

4. An agent for enhancing the antilipemic effect of an HMG-CoA reductase inhibitor, which contains methyl 1-(3,4-dimethoxyphenyl)-3-(3-ethylvaleryl)-4-hydroxy-6,7,8-trimethoxy-2-naphthoate, a glucuronic acid conjugate thereof, their salt or a solvate thereof.

5. Use of methyl 1-(3,4-dimethoxyphenyl)-3-(3-ethylvaleryl)-4-hydroxy-6,7,8-trimethoxy-2-naphthoate, a glucuronic acid conjugate thereof, their salt or a solvate thereof for enhancing the antilipemic effect of an HMG-CoA reductase inhibitor.

6. A preventive or therapeutic method for hyperlipemic disease, which comprises administering methyl 1-(3,4-dimethoxyphenyl)-3-(3-ethylvaleryl)-4-hydroxy-6,7,8-trimethoxy-2-naphthoate, a glucuronic acid conjugate thereof, their salt or a solvate thereof in combination with an HMG-CoA reductase inhibitor.
